# EUROPEAN PATENT SPECIFICATION

(11) **EP 4 006 384 B1**
(45) Date of publication and mention of the grant of the patent: **23.07.2025**
(21) Application number: 20849163.9
(22) Date of filing: 06.08.2020
(51) Int. Cl.: F16H 25/24, A61M 5/145, F16H 25/20

(54) **DRIVE DEVICE AND MEDICAL PUMP**
ANTRIEBSVORRICHTUNG UND MEDIZINISCHE PUMPE
DISPOSITIF D'ENTRAÎNEMENT ET POMPE MÉDICALE

(30) Priority: 07.08.2019 JP 2019145705
(43) Date of publication of application: 01.06.2022
(73) Proprietor: TERUMO Kabushiki Kaisha, Tokyo 151-0072 (JP)
(72) Inventor: HACHIMURA Shun, Ashigarakami-gun, Kanagawa 259-0151 (JP)
(74) Representative: Casalonga
(86) International application number: PCT/JP2020/030255
(87) International publication number: WO 2021/025132

(56) References cited:
- WO-A1-2018/046313
- JP-A- 2018 075 276
- JP-A- 2019 107 424
- JP-B2- S 542 110
- JP-U- S6 366 656

## Description

### Technical Field

The present invention relates to a drive device and a medical infusion pump. In particular, the present invention relates to a drive device according to the preamble of independent claim 1, such as it is e.g. known from WO 2018/04 6313 A1 or JP S63 66656U.

### Background Art

As a drive device forming a syringe pump, there is known a drive device including a feed screw that rotates about a central axis and a guided member that is guided in an axial direction along the central axis, the guided member including a nut member that has a screw portion screwed with the feed screw and a pressing portion that presses a plunger of a syringe. Since the guided member is moved in the axial direction by rotation of the feed screw in a state where the feed screw and the screw portion are screwed together, the plunger is pushed into a syringe body by the pressing portion of the guided member, whereby a liquid is extruded from the syringe body.

As such a drive device, for example, there is known a drive device, configured such that a screw portion is moved in a direction toward a central axis in a transverse section orthogonal to the central axis, as described in Patent Literature 1. According to such a configuration, even when a relative position between a feed screw and the screw portion in the traverse section varies due to thermal expansion of a component, a manufacturing error, or the like, the screw portion can move in the direction toward the central axis in the traverse section so as to narrow a gap between the feed screw and the screw portion caused by the variation, and thus, the misalignment of the screw portion with respect to the feed screw can be reduced. Therefore, it is possible to improve the accuracy of the movement of the guided member, that is, the plunger of the syringe in the axial direction.

### Citation List

### Patent Literature

Patent Literature 1: JP 2011-206376 A

### Summary of Invention

### Technical Problem

However, it is desirable that the guided member be movable with high accuracy by further reducing the misalignment of the screw portion with respect to the feed screw.

In view of such a point, an object of the present invention is to provide a drive device and a medical infusion pump capable of moving a guided member with high accuracy.

### Solution to Problem

A drive device according to a first aspect of the present invention includes a feed screw according to independent claim 1.

As one embodiment of the present invention, the drive device includes a pair of the nut members, and the screw portions of the pair of nut members are arranged so as to sandwich the feed screw.

As one embodiment of the present invention, in the drive device, each of the pair of nut members has a bearing portion that turns the screw portion such that the screw portion moves in the direction toward the central axis in the traverse section orthogonal to the central axis, each of a pair of the bearing portions has a long hole shape that is long in a direction intersecting the direction toward the central axis in the traverse section orthogonal to the central axis, and the guided member has a shaft member received by both of the bearing portions of the pair of nut members.

As one embodiment of the present invention, in the drive device, the guided member includes a housing to which the shaft member is attached and which accommodates the pair of nut members.

As one embodiment of the present invention, the drive device includes a switching member that switches a position of the screw portion between a screwing position where the screw portion is screwed with the feed screw and a non-screwing position where the screw portion is not screwed with the feed screw.

As one embodiment of the present invention, in the drive device, the switching member includes a cam that slides with respect to the nut member.

As one embodiment of the present invention, in the drive device, the cam slides with respect to a portion between the screw portion and the bearing portion in the nut member.

As one embodiment of the present invention, the drive device is arranged such that the cam is sandwiched between the pair of nut members.

As one embodiment of the present invention, the drive device includes a biasing member that biases the screw portion in a direction toward the central axis.

As one embodiment of the present invention, in the drive device, the guided member includes a spring forming the biasing member.

As one embodiment of the present invention, the drive device includes a position detection sensor that detects a position of the screw portion.

As one embodiment of the present invention, in the drive device, the position detection sensor includes a transmission unit that transmits a signal and a reception unit that receives the signal, and the nut member includes a blocking plate that blocks the signal when the nut member is located at one of the screwing position and the non-screwing position.

A medical infusion pump as a second aspect of the present invention includes the drive device.

As one embodiment of the present invention, the medical infusion pump includes a pressing portion in which the guided member presses a plunger of a syringe.

As one embodiment of the present invention, in the medical infusion pump, the switching member includes a cam that slides with respect to the nut member, a rotation shaft connected to the cam, and an operation member connected to the rotation shaft, the guided member includes an end member having the switching member and the pressing portion, and the operation member is rotatably supported by the end member.

### Advantageous Effects of Invention

According to the present invention, it is possible to provide the drive device and the medical infusion pump capable of moving the guided member with high accuracy.

### Brief Description of Drawings

Fig. 1 is a perspective view of a medical infusion pump according to an embodiment of the present invention.
Fig. 2 is a perspective view of a drive device of the medical infusion pump illustrated in Fig. 1.
Fig. 3 is an exploded perspective view of a part of the drive device illustrated in Fig. 2.
Fig. 4 is an exploded perspective view of two nut members and a cam illustrated in Fig. 3.
Fig. 5 is a transverse sectional view illustrating a part of the drive device illustrated in Fig. 3 in a state where a screw portion is at a non-screwing position.
Fig. 6 is a transverse sectional view illustrating a part of the drive device illustrated in Fig. 3 in a state where the screw portion is at a screwing position.
Fig. 7 is an explanatory view illustrating contour shapes bearing portions of the two nut members illustrated in Fig. 3 and a shaft member in a traverse section.

### Description of Embodiments

Hereinafter, a drive device and a medical infusion pump according to an embodiment of the present invention will be illustrated and described in detail with reference to the drawings.

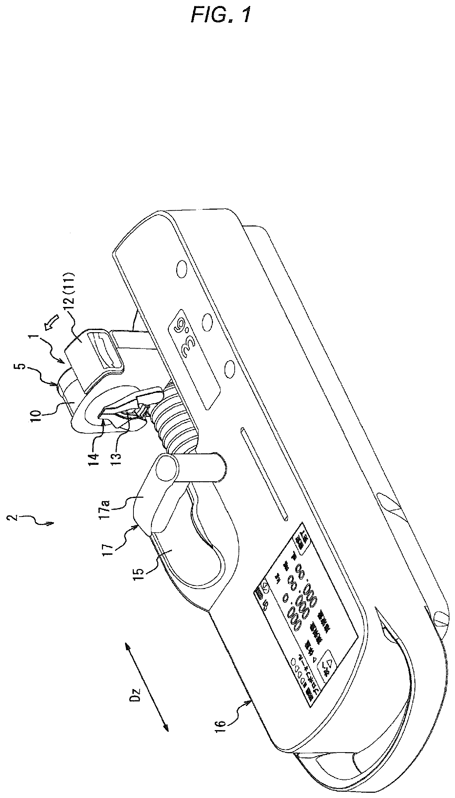

As illustrated in Fig. 1, a drive device 1 according to the present embodiment forms a medical infusion pump 2. The medical infusion pump 2 is a syringe pump, but is not limited thereto, and may be, for example, an infusion pump, a nutrient pump, a blood pump, or the like.

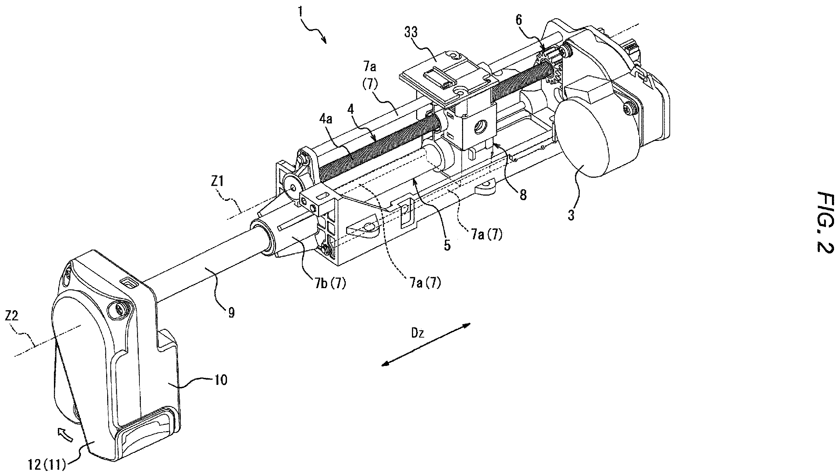

Fig. 2 illustrates the drive device 1 in a direction in which the up and down and the left and right are reversed with respect to Fig. 1. As illustrated in Fig. 2, the drive device 1 includes a drive source 3, a feed screw 4, and a guided member 5. The drive source 3 is configured using, for example, an electric motor, and generates power for rotating the feed screw 4. The drive source 3 rotates the feed screw 4 about a central axis Z1 via a power transmission device 6 configured using a gear train. The power transmission device 6 is not limited to the gear train. The feed screw 4 has the central axis Z1 and a male threaded portion 4a that spirally circles around the central axis Z1.

The guided member 5 is configured to be guided by a guide member 7 in an axial direction Dz along the central axis Z1. The guide member 7 includes three rod members 7a each extending in the axial direction Dz and a guide tube portion 7b. The guided member 5 includes a housing 8 that slides with respect to the three rod members 7a, and a hollow shaft 9 that slides with respect to the guide tube portion 7b. However, a structure for guiding the guided member 5 in the axial direction Dz is not limited thereto.

In addition, the guided member 5 includes an end member 10 and a switching member 11. One end of the hollow shaft 9 is connected to the housing 8, and the other end of the hollow shaft 9 is connected to the end member 10. The switching member 11 includes an operation member 12. The operation member 12 is supported by the end member 10 to be rotatable about a rotation axis Z2.

As illustrated in Fig. 1, the end member 10 includes a pressing portion 13 that presses a plunger of a syringe (not illustrated) in the axial direction Dz. In addition, the end member 10 is provided with a fixing member 14 including two arm members arranged to oppose the pressing portion 13 in the axial direction Dz. The fixing member 14 is configured to be open in conjunction with the operation member 12 to release the fixing of the plunger by rotating in a direction of the hollow arrow in Fig. 1 as the operation member 12 is operated, and to be closed in conjunction with the operation member 12 to fix the plunger as the operation member 12 is returned as illustrated in Fig. 1. An end flange of the plunger is sandwiched between the pressing portion 13 and the closed fixing member 14, whereby the plunger is fixed to the end member 10. The configuration of the fixing member 14 can be appropriately changed. A configuration in which the fixing member 14 is not provided may be also adopted.

The medical infusion pump 2 has a case 16 including a placement unit 15 on which a syringe body into which the plunger is pushed is placed. The syringe includes the syringe body and the plunger. A fixing lever 17 is provided in a portion adjacent to the placement unit 15. The fixing lever 17 is configured to rotate the fixing lever 17 to rotate an overhanging portion 17a of the fixing lever 17 to a position opposing the placement unit 15 as illustrated in Fig. 1 so as to fix the syringe body placed on the placement unit 15, and to rotate the fixing lever 17 to rotate the overhanging portion 17a of the fixing lever 17 to a position not opposing the placement unit 15 so as to release the fixing of the syringe body placed on the placement unit 15. The configuration of the fixing lever 17 can be changed as appropriate. A configuration in which the fixing lever 17 is not provided may be also adopted.

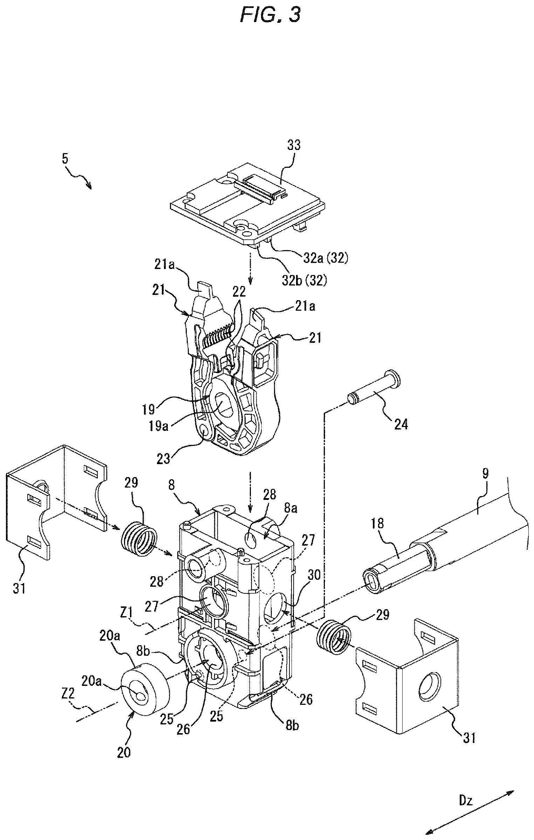

Fig. 3 illustrates a part of the guided member 5 of the drive device 1 in an exploded manner in a direction in which the left and right are reversed with respect to Fig. 2. As illustrated in Figs. 2 to 3, the switching member 11 includes the operation member 12, a cam shaft 18, a cam 19, and a ring member 20. The cam shaft 18 is a shaft member having the rotation axis Z2 as a central axis, and penetrates a hollow portion of the hollow shaft 9. One end of the cam shaft 18 is connected to the operation member 12 to be integrally rotatable. The cam 19 is connected to a portion protruding from the hollow shaft 9 on the other end side of the cam shaft 18 to be integrally rotatable. The ring member 20 is fixed to the other end of the cam shaft 18 by, for example, a fastener that is screwed into two screw holes 20a penetrating the ring member 20 in the radial direction and abuts on an outer peripheral surface of the cam shaft 18.

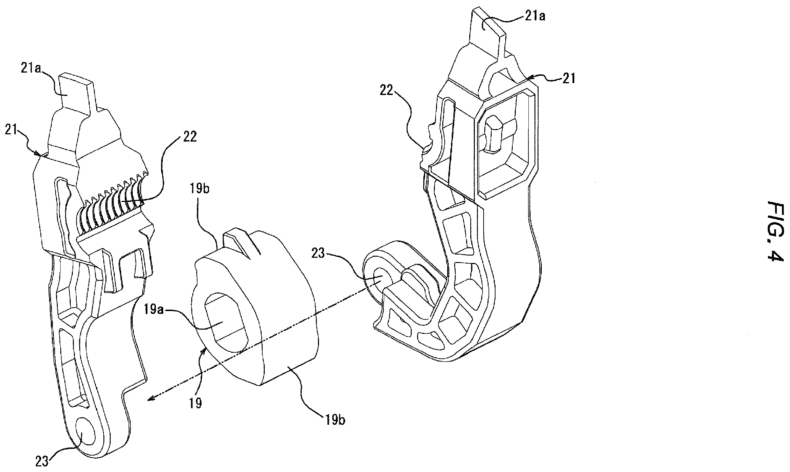

As illustrated in Figs. 3 to 4, the guided member 5 includes two nut members 21. Each of the nut members 21 has a screw portion 22 screwed with the male threaded portion 4a of the feed screw 4. Fig. 4 is an exploded perspective view of the two nut members 21 and the cam 19 illustrated in Fig. 3.

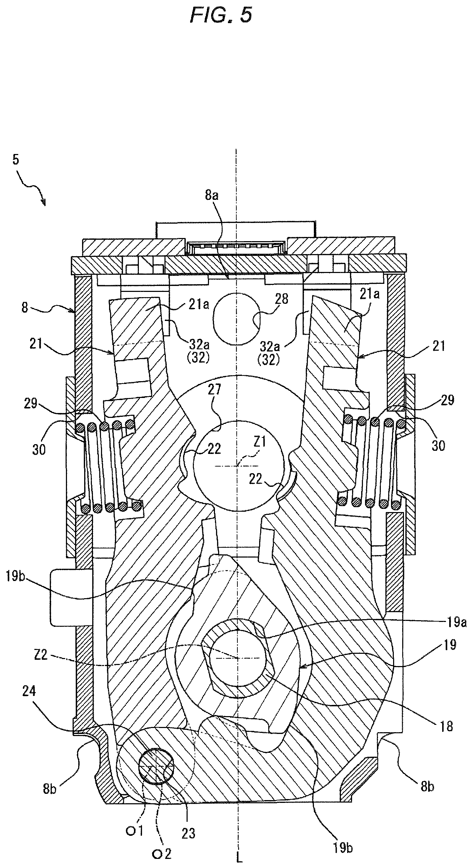

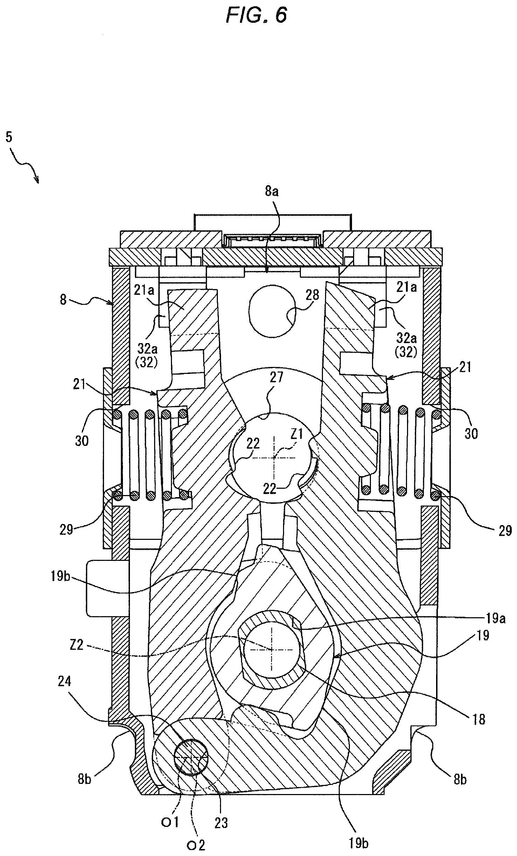

Fig. 5 is a transverse sectional view illustrating a part of the drive device 1 illustrated in Fig. 3 in a state where the screw portion 22 is at a non-screwing position. Fig. 6 is a transverse sectional view illustrating a part of the drive device 1 illustrated in Fig. 3 in a state where the screw portion 22 is at a screwing position. The non-screwing position is a position where the screw portion 22 is not screwed with the feed screw 4. The screwing position is a position where the screw portion 22 is screwed with the feed screw 4. The transverse section is a cross section orthogonal to the central axis Z1.

As illustrated in Figs. 5 to 6, the screw portions 22 of the two nut members 21 are arranged so as to sandwich the central axis Z1 of the feed screw 4. In addition, each of the nut members 21 has a bearing portion 23 that turns the screw portion 22 such that the screw portion 22 moves in a direction toward the central axis Z1 in the traverse section. The guided member 5 has a shaft member 24 that is received by both of the bearing portions 23 of the two nut members 21.

The cam 19 is arranged to be sandwiched between the two nut members 21 so as to slide with respect to each of portions between the screw portion 22 and the bearing portion 23 in the two nut members 21.

As illustrated in Fig. 3, the housing 8 has a box shape having an open portion 8a, formed by an opening into which the two nut members 21 sandwiching the cam 19 can be inserted, on an upper surface in Fig. 3, and accommodates the two nut members 21. However, the housing 8 may be changed to a member having a shape that does not accommodate the two nut members 21. Each of two surfaces of the housing 8 opposing each other in the axial direction Dz has a shaft member through-hole 25 through which the shaft member 24 passes. The shaft member 24 is configured using a headed pin. In a state where the cam 19 is sandwiched between the two nut members 21 and accommodated in the housing 8, the shaft member 24 is passed through the two shaft member through-holes 25 of the housing 8 and the bearing portions 23 of the two nut members 21, and a C-ring-shaped fastener is fitted to a distal end of the shaft member 24, whereby the shaft member 24 is attached to the housing 8. An attachment structure for attaching the shaft member 24 to the housing 8 can be appropriately changed.

Each of the two surfaces of the housing 8 opposing each other in the axial direction Dz has a cam shaft through-hole 26 through which the cam shaft 18 passes. A region on the other end side including the other end of the cam shaft 18 has a non-circular cross-sectional shape fitted with a cam hole 19a penetrating the cam 19. In the state where the cam 19 is sandwiched between the two nut members 21 and accommodated in the housing 8, the region on the other end side of the cam shaft 18 passes through the two cam shaft through-holes 26 of the housing 8 and the cam hole 19a of the cam 19, and the ring member 20 is fixed to the other end of the cam shaft 18, whereby the cam shaft 18 is attached to the housing 8. An attachment structure for attaching the cam shaft 18 to the housing 8 can be appropriately changed.

In addition, each of the two surfaces of the housing 8 opposing each other in the axial direction Dz has a feed screw through-hole 27 through which the feed screw 4 passes and a rod member through-hole 28 through which one of the three rod members 7a passes. A sliding-contact portion 8b is provided on each of outer surfaces of two corner portions of the housing 8 located at the lower right and the lower left in Fig. 3. The two sliding-contact portions 8b are in sliding contact with the remaining two of the three rod members 7a, respectively.

The drive device 1 includes two coiled springs 29 as biasing members that bias the two screw portions 22 in the direction toward the central axis Z1. Each of two surfaces of the housing 8 opposing each other in the left-right direction in Fig. 3 has a spring through-hole 30 through which the spring 29 passes. Each of the two springs 29 biases the screw portion 22 in the direction toward the central axis Z1. In the state where the cam 19 is sandwiched between the two nut members 21 and accommodated in the housing 8, each of the two springs 29 passes through the spring through-hole 30, and the cover 31 is fitted to the housing 8, whereby the two springs 29 are arranged. A structure for arranging the two springs 29 can be appropriately changed. The two springs 29 are not limited to the coil shape. The biasing member is not limited to the two springs 29. A configuration in which the drive device 1 has no biasing member may be adopted.

The switching member 11 slides with respect to the two nut members 21 as the cam 19 rotates about the rotation axis Z2, thereby switching the positions of the two screw portions 22 between the screwing position illustrated in Fig. 6 and the non-screwing position illustrated in Fig. 5. In Fig. 5, the positions of the two screw portions 22 are switched from the screwing position to the non-screwing position as the cam 19 rotates counterclockwise.

When each of the screw portions 22 is at the screwing position, the guided member 5 can be moved in the axial direction Dz by rotating the feed screw 4 using the drive source 3, and thus, the plunger can be pushed into the syringe body by the pressing portion 13, and a liquid can be extruded from the syringe body. When each of the screw portions 22 is at the non-screwing position, a user can grip the guided member 5 and freely move the guided member 5 in the axial direction Dz, and thus, the plunger can be easily attached to the pressing portion 13 of the guided member 5 when the syringe having a desired dimension is attached to the medical infusion pump 2.

In addition, each of the screw portions 22 moves in the direction toward the central axis Z1 when moving from the non-screwing position toward the screwing position. Therefore, even when a relative position between the feed screw 4 and each of the screw portions 22 in the traverse section varies due to thermal expansion of a component, a manufacturing error, or the like, each of the screw portions 22 can move in the direction toward the central axis Z1 in the traverse section so as to narrow a gap between the feed screw 4 and each of the screw portions 22 caused by the variation, and thus, the misalignment of each of the screw portions 22 with respect to the feed screw 4 can be reduced. Therefore, it is possible to improve the accuracy of movement of the guided member 5, that is, the plunger of the syringe in the axial direction Dz.

Further, the drive device 1 of the present embodiment is configured such that each of the screw portions 22 also moves in a direction intersecting the direction toward the central axis Z1 in order to further reduce the misalignment of each of the screw portions 22 with respect to the feed screw 4 to move the guided member 5 with high accuracy. More specifically, the bearing portion 23 of each of the nut members 21 has a long hole shape that is long in a direction substantially orthogonal to the direction in which the screw portion 22 is directed toward the central axis Z1 in the traverse section. That is, each of the bearing portions 23 has a short axis O1 and a long axis O2 orthogonal to each other, and the long axis O2 extends in the direction (vertical direction in Fig. 6) substantially orthogonal to the direction in which the screw portion 22 is directed toward the central axis Z1.

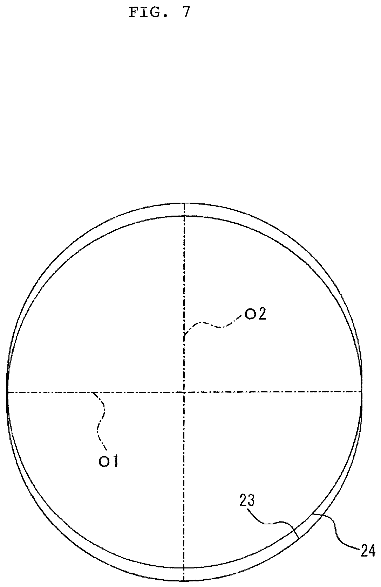

Fig. 7 illustrates contour shapes of the bearing portions 23 of the two nut members 21 illustrated in Fig. 3 and the shaft member 24 in the traverse section. As illustrated in Fig. 7, a contour of the shaft member 24 is substantially a perfect circular shape, and the contour of the bearing portion 23 is a substantially oval shape having the short axis O1 and the long axis O2, that is, a shape in which half arcs are connected by a straight line.

However, the contour shapes of the shaft member 24 and the bearing portion 23 are not limited thereto. In addition, an extending direction of the long axis O2 is not limited to the direction substantially orthogonal to the direction in which the screw portion 22 is directed toward the central axis Z1, and it is sufficient that the extending direction of the long axis O2 intersects with the direction in which the screw portion 22 is directed toward the central axis Z1. With such a configuration, each of the screw portions 22 can be moved not only in a direction toward the central axis Z1 but also in a direction intersecting the direction, and the misalignment of each of the screw portions 22 with respect to the feed screw 4 can be further reduced.

In addition, the bearing portions 23 of the two nut members 21 can be individually moved with respect to the shaft member 24 by, for example, raising one bearing portion 23 and lowering the other bearing portion 23, and thus it is possible to further reduce the misalignment of each screw portion 22 with respect to the feed screw 4.

The short axis O1 has a length corresponding to "gap fitting" (for example, light fitting or fitting in JIS B 0401-1, 0401-2 (1998)) which is general fitting that allows a component to be relatively movable with respect to the shaft member 24, and the long axis O2 preferably has a length exceeding this length. For example, a gap of several tens of microns can be set in a direction along the short axis O1, and a gap of several hundreds of microns can be set in a direction along the long axis O2. With such a setting, each of the screw portions 22 can be moved not only in the direction toward the central axis Z1 but also in the direction intersecting the direction, so that the misalignment of each of the screw portions 22 with respect to the feed screw 4 can be further reduced. In addition, when the position of each of the screw portions 22 is switched to the non-screwing position by the cam 19, the movement of each of the bearing portions 23 is made difficult, so that each of the screw portions 22 can be smoothly moved in the direction away from the central axis Z1.

The cam 19 has two cam surfaces 19b opposing each other across the rotation axis Z2. Each of the cam surfaces 19b slides with respect to a portion between the screw portion 22 and the bearing portion 23 in each of the nut members 21. As illustrated in Fig. 5, one nut member 21 in sliding contact with one cam surface 19b closer to the central axis Z1 has a shorter overall length than the other nut member 21. That is, the bearing portion 23 of the one nut member 21 is arranged on a side where the screw portion 22 of the one nut member 21 is located with respect to a straight line L passing through the central axis Z1 and the rotation axis Z2 in the traverse section. That is, the bearing portions 23 of the two nut members 21 and the shaft member 24 are arranged on the left side of the straight line L in Fig. 5. With such a setting, the balance of a force when the two nut members 21 are pushed and opened by the cam 19 is improved, and the positions of the two screw portions 22 can be smoothly switched. However, the arrangement of the bearing portion 23 and the shaft member 24 is not limited thereto.

For example, the two nut members 21, the cam 19, and the housing 8 may be made of a synthetic resin, and the feed screw 4, the shaft member 24, the cam shaft 18, and the ring member 20 may be made of metal. Since the synthetic resin is used instead of metal, the manufacturing cost of a member can be reduced, and weight reduction or the like can be achieved. Examples of the synthetic resin forming the two nut members 21, the cam 19, and the housing 8 include polyether ether ketone (PEEK) and polyphenylene sulfide (PPS). In the case where the two nut members 21, the cam 19, and the housing 8 are made of the synthetic resin as described above, however, the relative position between the feed screw 4 and the screw portion 22 in the traverse section easily varies due to a difference in linear thermal expansion coefficient between components accompanying a change in ambient temperature as compared with a case where these are made of metal. In addition, in a case where the two nut members 21 and the like are formed by injection molding using a synthetic resin, variations in dimensions of individual members are large as compared with a case of formation by cutting using metal. Therefore, the configuration in which the screw portions 22 are moved in both the direction toward the central axis z1 and the direction intersecting the direction in the traverse section as in the present embodiment is particularly suitable in a case of adopting the above material configuration.

As illustrated in Figs. 3, 5, and 6, the drive device 1 includes two position detection sensors 32 that detect the positions of the screw portions 22. Each of the position detection sensors 32 includes, for example, a transmission unit 32a that transmits a signal such as light and a reception unit 32b that receives the signal. Each of the nut members 21 has a blocking plate 21a that blocks the signal when the nut member 21 is located at one of the screwing position and the non-screwing position. According to such a configuration, a screwing state of the screw portion 22 can be monitored, and thus, safety can be improved.

The two position detection sensors 32 are mounted on a substrate 33. As illustrated in Figs. 2 to 3, the substrate 33 is attached to the housing 8 such that the two position detection sensors 32 are located inside the housing 8 and the substrate 33 is arranged to cover the open portion 8a of the housing 8. The number of the position detection sensors 32 and the blocking plates 21a is not limited to two, and may be one. A configuration in which the position detection sensor 32 and the blocking plate 21a are not provided may be adopted.

The above-described embodiment is merely an example of embodiments of the present invention, and it is a matter of course that various modifications can be made without departing from the scope of the invention as defined in the accompanying claims.

The two nut members 21 are configured to be rotatably supported by the common shaft member 24 in the above-described embodiment, but may be configured to be rotatably supported by individual shafts without being limited thereto. Although the two nut members 21 are provided in the above-described embodiment, the number of the nut members 21 is not limited thereto, and may be, for example, one.

Although the nut member 21 has the bearing portion 23 supported by the shaft member 24 in the above-described embodiment, the present invention is not limited thereto, and may be configured such that the nut member 21 has a shaft supported by a bearing portion. Although the nut member 21 is configured to rotate such that the screw portion 22 moves in the direction toward the central axis Z1 in the traverse section in the above-described embodiment, the present invention is not limited thereto, and for example, the nut member 21 may be configured to move in parallel such that the screw portion 22 moves in the direction toward the central axis Z1 in the traverse section. In this case, the nut member 21 can be configured to move also in a direction intersecting the direction of the above-described parallel movement such that the screw portion 22 also moves in the direction intersecting the direction toward the central axis Z1 in the traverse section.

Although the switching member 11 includes the cam 19 in the above-described embodiment, the present invention is not limited thereto. In addition, a configuration in which the drive device 1 does not include the switching member 11 may be adopted.

### Reference Signs List

- 1: drive device
- 2: medical infusion pump
- 3: drive source
- 4: feed screw
- 4a: male threaded portion
- 5: guided member
- 6: power transmission device
- 7: guide member
- 7a: rod member
- 7b: guide tube portion
- 8: housing
- 8a: open portion
- 9: hollow shaft
- 10: end member
- 11: switching member
- 12: operation member
- 13: pressing portion
- 14: fixing member
- 15: placement unit
- 16: case
- 17: fixing lever
- 17a: overhanging portion
- 18: cam shaft
- 19: cam
- 19a: cam hole
- 19b: cam surface
- 20: ring member
- 20a: screw hole
- 21: nut member
- 21a: blocking plate
- 22: screw portion
- 23: bearing portion
- 24: shaft member
- 25: shaft member through-hole
- 26: cam shaft through-hole
- 27: feed screw through-hole
- 28: rod member through-hole
- 29: spring
- 30: spring through-hole
- 31: cover
- 32: position detection sensor
- 32a: transmission unit
- 32b: reception unit
- 33: substrate
- Z1: central axis
- Z2: rotation axis
- Dz: axial direction
- O1: short axis
- O2: long axis
- L: straight line

## Claims

1. A drive device (1) comprising:
a feed screw (4) that is rotatable about a central axis (Z1); and
a guided member (5) guidable in an axial direction (Dz) along the central axis (Z1),
wherein the guided member (5) includes a nut member (21) having a screw portion (22) configured to be screwed with the feed screw (4),
the screw portion (22) is configured to move in both a direction toward the central axis (Z1) and a direction intersecting the direction in a transverse section orthogonal to the central axis (Z1),
**characterized in that**
the nut member (21) has a bearing portion (23) that is configured to turn the screw portion (22) such that the screw portion (22) moves in the direction toward the central axis (Z1) in the transverse section orthogonal to the central axis (Z1), and
the bearing portion (23) has a long hole shape that is long in a direction intersecting the direction in which the screw portion (22) is directed toward the central axis (Z1) in the transverse section orthogonal to the central axis (Z1).

2. The drive device (1) according to claim 1, comprising
a pair of the nut members (21),
wherein the screw portions (22) of the pair of nut members (21) are configured to be arranged so as to sandwich the feed screw (4).

3. The drive device (1) according to claim 2, wherein
each of the pair of nut members (21) has a bearing portion (23) that is configured to turn the screw portion (22) such that the screw portion (22) moves in the direction toward the central axis (Z1) in the traverse section orthogonal to the central axis (Z1),
each of a pair of the bearing portions (23) has a long hole shape that is long in a direction intersecting the direction toward the central axis (Z1) in the traverse section orthogonal to the central axis (Z1), and
the guided member (5) has a shaft member (24) configured to be received by both of the bearing portions (23) of the pair of nut members (21).

4. The drive device (1) according to claim 3, wherein
the guided member (5) includes a housing (8) to which the shaft member (24) is configured to be attached and which accommodates the pair of nut members (21).

5. The drive device (1) according to any one of claims 1 to 4, further comprising
a switching member (11) that is configured to switch a position of the screw portion (22) between a screwing position where the screw portion (22) is screwed with the feed screw (4) and a non-screwing position where the screw portion (22) is not screwed with the feed screw (4).

6. The drive device (1) according to claim 5, wherein
the switching member (11) has a cam (19) that is configured to slide with respect to the nut member (21).

7. A medical infusion pump (2) comprising
the drive device (1) according to any one of claims 1 to 6.

8. The medical infusion pump (2) according to claim 7, wherein
the guided member (5) has a pressing portion (13) that is configured to press a plunger of a syringe.

## Patentansprüche

1. Antriebsvorrichtung (1), umfassend:
eine Vorschubschraube (4), die um eine Mittelachse (Z1) drehbar ist; und
ein geführtes Element (5), das in axialer Richtung (Dz) entlang der Mittelachse (Z1) führbar ist,
wobei das geführte Element (5) ein Mutternelement (21) umfasst, das einen Schraubenabschnitt (22) aufweist, der so konfiguriert ist, dass er mit der Vorschubschraube (4) verschraubt werden kann,
wobei der Schraubenabschnitt (22) so konfiguriert ist, dass er sich sowohl in Richtung zu der Mittelachse (Z1) als auch in einer Richtung bewegt, welche die Richtung in einem Querschnitt orthogonal zu der Mittelachse (Z1) schneidet,
**dadurch gekennzeichnet, dass**
das Mutternelement (21) einen Lagerabschnitt (23) aufweist, der so konfiguriert ist, dass er den Schraubenabschnitt (22) derart dreht, dass sich der Schraubenabschnitt (22) in der Richtung zur Mittelachse (Z1) in dem Querschnitt orthogonal zu der Mittelachse (Z1) bewegt, und
der Lagerabschnitt (23) eine Langlochform aufweist, die in einer Richtung lang ist, welche die Richtung schneidet, in welcher der Schraubenabschnitt (22) in dem Querschnitt orthogonal zu der Mittelachse (Z1) auf die Mittelachse (Z1) gerichtet ist.

2. Antriebsvorrichtung (1) nach Anspruch 1, umfassend ein Paar der Mutternelemente (21),
wobei die Schraubenabschnitte (22) des Paares von Mutterelementen (21) so angeordnet sind, dass sie die Vorschubschraube (4) zwischen sich einklemmen.

3. Antriebsvorrichtung (1) nach Anspruch 2, wobei
jedes von dem Paar der Mutternelemente (21) einen Lagerabschnitt (23) aufweist, der so konfiguriert ist, dass er den Schraubenabschnitt (22) derart dreht, dass sich der Schraubenabschnitt (22) in der Richtung zur Mittelachse (Z1) in dem Querschnitt orthogonal zu der Mittelachse (Z1) bewegt,
jeder der beiden Lagerabschnitte (23) eine Langlochform aufweist, die in einer Richtung lang ist, welche die Richtung auf die Mittelachse (Z1) zu in dem Querschnitt orthogonal zu der Mittelachse (Z1) schneidet, und
das geführte Element (5) ein Schaftelement (24) aufweist, das so konfiguriert ist, dass es von beiden Lagerabschnitten (23) des Paares von Mutterelementen (21) aufgenommen werden kann.

4. Antriebsvorrichtung (1) nach Anspruch 3, wobei
das geführte Element (5) ein Gehäuse (8) umfasst, wobei das Schaftelement (24) so konfiguriert ist, dass es daran befestigt werden kann, und es das Paar von Mutternelementen (21) aufnimmt.

5. Antriebsvorrichtung (1) nach einem der Ansprüche 1 bis 4, ferner umfassend
ein Schaltelement (11), das so konfiguriert ist, dass es eine Position des Schraubenabschnitts (22) zwischen einer Schraubposition, in welcher der Schraubenabschnitt (22) mit der Vorschubschraube (4) verschraubt ist, und einer Nicht-Schraubposition, in welcher der Schraubenabschnitt (22) nicht mit der Vorschubschraube (4) verschraubt ist, umschaltet.

6. Antriebsvorrichtung (1) nach Anspruch 5, wobei
das Schaltelement (11) einen Nocken (19) aufweist, der so konfiguriert ist, dass er in Bezug auf das Mutternelement (21) gleitet.

7. Medizinische Infusionspumpe (2), umfassend
die Antriebsvorrichtung (1) nach einem der Ansprüche 1 bis 6.

8. Medizinische Infusionspumpe (2) nach Anspruch 7, wobei
das geführte Element (5) einen Druckabschnitt (13) aufweist, der so konfiguriert ist, dass er einen Kolben einer Spritze drückt.

## Revendications

1. Dispositif d'entraînement (1) comprenant :
une vis d'alimentation (4) qui est rotative autour d'un axe central (Z1) ; et
un organe guidé (5) guidable dans une direction axiale (Dz) le long de l'axe central (Z1),
dans lequel l'organe guidé (5) comporte un organe d'écrou (21) présentant une partie de vis (22) configurée pour être vissée avec la vis d'alimentation (4),
la partie de vis (22) est configurée pour se déplacer à la fois dans une direction vers l'axe central (Z1) et dans une direction croisant la direction dans une section transversale orthogonale à l'axe central (Z1),
**caractérisé en ce que**
l'organe d'écrou (21) présente une partie d'appui (23) qui est configurée pour tourner la partie de vis (22) de sorte que la partie de vis (22) se déplace dans la direction vers l'axe central (Z1) dans la section transversale orthogonale à l'axe central (Z1), et
la partie d'appui (23) présente une forme de trou long qui est long dans une direction croisant la direction dans laquelle la partie de vis (22) est dirigée vers l'axe central (Z1) dans la section transversale orthogonale à l'axe central (Z1).

2. Dispositif d'entraînement (1) selon la revendication 1, comprenant
une paire des organes d'écrou (21),
dans lequel les parties de vis (22) de la paire d'organes d'écrou (21) sont configurées pour être agencées de manière à intercaler la vis d'alimentation (4).

3. Dispositif d'entraînement (1) selon la revendication 2, dans lequel
chacune de la paire d'organes d'écrou (21) présente une partie d'appui (23) qui est configurée pour tourner la partie de vis (22) de sorte que la partie de vis (22) se déplace dans la direction vers l'axe central (Z1) dans la section transversale orthogonale à l'axe central (Z1), chacune d'une paire des parties d'appui (23) présente une forme de trou long qui est long dans une direction croisant la direction vers l'axe central (Z1) dans la section transversale orthogonale à l'axe central (Z1), et l'organe guidé (5) présente un organe de tige (24) configuré pour être reçu par les deux parties d'appui (23) de la paire d'organes d'écrou (21).

4. Dispositif d'entraînement (1) selon la revendication 3, dans lequel
l'organe guidé (5) comporte un logement (8) auquel l'organe de tige (24) est configuré pour être fixé et qui reçoit la paire d'organes d'écrou (21).

5. Dispositif d'entraînement (1) selon l'une quelconque des revendications 1 à 4, comprenant en outre
un organe de commutation (11) qui est configuré pour commuter une position de la partie de vis (22) entre une position de vissage où la partie de vis (22) est vissée avec la vis d'alimentation (4) et une position de non-vissage où la partie de vis (22) n'est pas vissée avec la vis d'alimentation (4).

6. Dispositif d'entraînement (1) selon la revendication 5, dans lequel
l'organe de commutation (11) présente une came (19) qui est configurée pour coulisser par rapport à l'organe d'écrou (21).

7. Pompe à perfusion médicale (2) comprenant
le dispositif d'entraînement (1) selon l'une quelconque des revendications 1 à 6.

8. Pompe à perfusion médicale (2) selon la revendication 7, dans laquelle
l'organe guidé (5) présente une partie de pressage (13) qui est configurée pour presser un piston d'une seringue.
